# EUROPEAN PATENT SPECIFICATION

(11) **EP 1 265 552 B1**
(45) Date of publication and mention of the grant of the patent: **10.01.2007**
(21) Application number: 01931537.3
(22) Date of filing: 19.03.2001
(51) Int. Cl.: A61F 2/06, A61B 17/32, A61M 25/06

(54) **SYSTEM FOR BYPASSING AN ARTERY BLOCK**
SYSTEM ZUR ÜBERBRÜCKUNG EINER ARTERIELLEN BLOCKIERUNG
SYSTEME SERVANT A EFFECTUER UN PONTAGE ARTERIEL

(30) Priority: 20.03.2000 SE 0000900
(43) Date of publication of application: 18.12.2002
(73) Proprietor: Abbott Laboratories Vascular Enterprises Limited, Terrace, Dublin 2 (IE)
(72) Inventor: Solem, Jan Otto, 8234 Stetten (CH)
(74) Representative: Giver, Sören Bo
(86) International application number: PCT/EP2001/003104
(87) International publication number: WO 2001/070133

(56) References cited:
- WO-A-97/13463
- WO-A-99/35978
- WO-A-99/35980
- US-A- 5 830 222
- US-A- 5 989 276

## Description

### Background of the invention

The present invention is related to a catheter system for use in bypassing a block or occlusion in an artery.

Coronary artery disease is the most common cause of death today. Apart from that, more people suffer from pain and discomfort from the disease than die. The cause of these problems is mostly localised blocks, also called stenosis, in the arteries that support the heart muscle with energy and oxygen. Pain or death of muscle tissue occurs when this support ceases or is insufficient.

Today almost all incomplete blocks in these arteries are treated by means of dilating balloons that are placed across the blocks and inflated, whereby the blocked site of the vessel may be widened and the arterial fresh blood may pass again. The irregularities that occur in the vessel wall after such percutaneous transarterial coronary angioplasty (PTCA) are today secured and controlled by means of a so called stent, e.g. a metal cylinder that also is dilated in the same way when it is in place over the treated area of the vessel, whereby it is pressed against the vessel wall and will remain there as a support.

The vessel has, however, to be open through the narrow part to enable treatment of the block with a balloon. This is the case for about half the population of the coronary deceased people. The other half will have to undergo coronary artery bypass surgery. During this procedure the stop in the vessel is shunter (or bypassed) by means of a conduit usually taken from the person himself whereby fresh blood is guided from another artery upstream through a new passage past the blocked area into the distal part of such a deceased vessel.

Every year about one million people in the western world undergo the balloon treatment and another million people have coronary artery bypass surgery. The surgery usually involves the use of extra-corporeal circulation, the heart and lung machine, full anaesthesia, open chest and long recovery and rehabilitation times after the procedure. Having had a PTCA, however, the patient will leave the hospital the following day since no anaesthesia was necessary and also no big surgery was done.

According to U. S. Patent No. 5,830,222 of Joshua Makower, a percutaneous revascularization is obtained by arterializing a distal part of a vein, whereby blood flows from a blocked artery and in a retrograde fashion in this distal part of the vein, or by simply using a portion of the vein as a bypass graft, the part of the vein distal to this portion is out off. Thus, in both these cases the ordinary function of the distal part of the vein is blocked which may lead to problems as regards blood drainage from the area of the cut-off distal part of the vein.

### Summary of the invention

A main object of the present invention is to provide a new possibility for many of the patients that undergo surgery today to be treated with a similar method as the PTCA, i.e. awake and with no anaesthesia and no big surgery even if the artery in question is totally blocked.

A further object of the invention is to provide such surgery without eliminating the function of parts of veins distal to the block or occlusion in an artery.

This object is attained on the basis of the anatomical peculiarity that most of the arteries in the human body are in a very close proximity a vein draining blood from the same area as the artery supports.

In the case of the heart, the artery and the vein usually are so close to each other that contact is present. Moreover, it is very easy to get access to the venous system of the heart since the coronary sinus, the endpoint of the venous drainage from the heart, is easily accessible in the right atrium, only about 20 cm from the patient's neck and is connected to the big jugular vein in the neck.

Further, an observation that is of great importance for the present invention is the fact that the veins are usually much bigger in diameter than the arteries. According to the invention, a covered stent of smaller diameter than the vein is used to bypass the block in the adjacent artery without blocking the flow in the vein itself. Thus the covered stent is positioned in the vein and its ends are connected to the adjacent artery on either side of the block therein.

In this context, the covered stent is essentially equivalent with a vascular graft or a scented vascular graft. In this application, a covered stent is to be understood as a vessel which is flexible, may be dilated and will maintain its dilated shape.

More precisely, the above objects of the invention are obtained by a catheter system as claimed in the subsequent independent claim. Preferred embodiments of the invention are defined in the dependent claims.

Here after a method will be disclosed, which discloses some of the advantages obtainable of the inventive catheter system.

A method of bypassing a blocking in an artery which extends along a vein, comprises the steps of forming a first connection between said artery and said vein proximal to the blocking in the artery, forming a second connection between said artery and said vein distal to the blocking in the artery, introducing covered stent through said artery proximal to the blocking therein, through said first connection into said vein, via said vein to and through said second connection, and into said artery distal to the blocking therein, such that a proximal end of the covered stent is positioned in the artery proximal to the blocking therein and a distal end of the covered stent is positioned in the artery distal to the blocking therein, and fixing the proximal and distal ends of the covered stent within the artery.

The forming of said first connection preferably comprises introducing a first catheter through the artery, said first catheter having a lateral opening at a distal end thereof, positioning said lateral opening so as to face said vein proximal to said blocking, introducing a first therapeutic wire through said first catheter, said first therapeutic wire having an active distal end, advancing the distal end of the first therapeutic wire through the lateral opening of said first catheter, out of the artery and into the vein, thereby forming the first connection.

The forming of the second connection preferably comprises introducing a second catheter through the vein, said second catheter having a lateral opening at a distal end thereof, positioning said lateral opening so as to face said artery distal to said blocking, introducing a second therapeutic wire through said second catheter, said second therapeutic wire having an active distal end, advancing the distal end of the second, therapeutic wire through the lateral opening of said second catheter, out of the vein and into the artery distal to said blocking, thereby forming the second connection.

Most preferable is to include a step of catching the distal end of the first therapeutic wire and retracting it back through the vein, the first therapeutic wire then extending through the artery proximal to the blocking, through the first connection and through the vein proximal to the blocking. Hereby, proximal ends of the first and second therapeutic wires extending through the vein, may be joined to each other, preferably outside the body, and the first therapeutic wire may be retracted through the artery, such that the second therapeutic wire extends from the artery distal to the blocking through the second connection, via the vein to and through the first connection and into the artery proximal to the blocking, and finallys along the artery proximal of the blocking.

Concluding, the covered stent could be introduced on the second therapeutic wire for bypassing the blocking and be fixed by dilation of the stent, preferably by means of a balloon.

It should be noted that the stent be dilated to a diameter (or cross-sectional area), which is smaller than the diameter (or cross-sectional area) of the vein enclosing part of the covered stent. Hereby, additional free lumen is maintained in the vein for blood flow in the vein itself.

In order to position the lateral opening of said first catheter so as to face said vein proximal to said blocking, a detector may be introduced into the vein for detecting the position, of the lateral opening in the first catheter. This detector is used first to detect the position of the blocking in the artery and then to detect the position of the lateral opening in the first catheter.

The position detector may be an ultrasonic transducer, which is used to detect the position of an ultrasound reflecting marking, provided on first catheter in a predetermined relation to the lateral opening at the distal end thereof. However, any other type of position indication technique known in the art may be used, such as MRI, OCT, angiography and radiography.

The position detector is preferably disposed on the second catheter and may also be used to position the lateral opening of said second catheter so as to face said artery distal to said blocking. The detector is preferably disposed near the lateral opening thereof for detecting the position of the artery distal to the blocking.

The position detector may have the capacity of viewing in a more limited sector and/or 360° around the catheter. Further, the position detector should be able to penetrate and depict structure outside the vessel of its own location, i.e. beyond the wall of that vessel and into adjacent tissue or vessels.

A method of bypassing a blocking in an artery which extends along a vein, alternatively may comprise the steps of providing a covered stent having a proximal end and a distal end, positioning the covered stent within said vein having its proximal end introduced into said artery proximal to the blocking therein and having its distal end introduced into to said artery distal to the blocking therein, connecting the proximal end of the covered stent to the artery proximal to the blocking, and connecting the distal end of the covered stent to the artery distal to the blocking,

The covered stent used should preferably have a diameter, which is smaller than the diameter of the vein enclosing part of the covered stent.

The catheter system of the invention for use in bypassing a blocking in an artery and performing the above method preferably comprises four components. These components are an arterial catheter, an intravenous catheter, a guide-wire system, and finally a covered stent used as graft.

According to the invention the catheter system comprises an arterial catheter for introduction into said artery, a distal end of the arterial catheter having a lateral opening to be positioned proximal to the blocking in said artery, and a wire having a cutting tip at a distal end thereof, said wire being advancable through the arterial catheter so as to project the cutting tip out through the lateral opening at the distal end of the arterial catheter and thereby laterally out through a wall of the artery,

The arterial catheter may have a flap connected to a distal edge of the lateral opening, said flap being inclined towards the opposite side of the arterial catheter and having a free end proximal of said distal edge. Preferably, the flap is flexible to a position substantially covering the lateral opening of the arterial catheter.

The catheter system may use ultrasound or other technique for the determination of position. An ultra-sound reflecting material may be fixed in a predetermined position relative to the lateral opening of the arterial catheter. Preferably, this ultrasound reflecting material at least partly encircles the lateral opening of the arterial catheter.

The catheter system further may comprise an ultrasound catheter for introduction into a vein extending along said artery which ultrasound catheter may comprise an ultrasonic transducer for determining the position of the blocking in the artery and for monitoring a positioning of the lateral opening of the arterial catheter when introduced into said artery so as to face the vein.

Preferably, the ultrasonic transducer is directed laterally substantially in the direction of the laterally directed distal opening and positioned distal to the laterally directed opening. Alternative catheters employing other imaging modalities may also be used.

It should be noted that the invention may be used for any pair of an artery and an adjacent vein in the body and not exclusively for such pairs in the heart.

### Short description of the drawings

Fig. 1 illustrates the anatomy of a human heart.
Fig. 2 illustrates a finished coupling according to the present invention bypassing a blocking in an artery of the heart.
Fig. 3 is a top plan view of a distal part of an embodiment of a first catheter designed as an arterial catheter according to the invention for use in a method or bypassing a blocking in an artery.
Fig. 4 is a longitudinal sectional view of the first catheter shown in Fig. 3.
Fig. 5 is an elevation view of the first catheter shown in Fig. 3 and also illustrates a wire introduced through a distal end opening of the catheter.
Fig. 6 is an elevation view of the first catheter shown in Fig. 3 and also illustrates a wire having an active head introduced from the proximal end of the catheter and out of a lateral opening of the catheter.
Figs. 7 and 8 illustrate two positions of a flap device of the first catheter.
Fig. 9 is a top plan view of a distal part of a first embodiment of a second catheter having two lumens and being designed as an intravenous ultrasound catheter according to the invention for use in a method of bypassing a blocking in an artery.
Fig. 10 is an elevation view of the first embodiment of the second catheter shown in Fig. 9
Fig. 11 is an elevation view of the first embodiment of the second catheter shown in Fig. 9 and also illustrates two wires introduced through the lumens thereof.
Fig. 12 is a top plan view of a distal part of a second embodiment of a second catheter having a single lumen and being designed as an intravenous ultrasound catheter according to the invention for use in a method of bypassing a blocking in an artery.
Fig. 13 is a longitudinal sectional view of the second embodiment of the second catheter shown in Fig. 12.
Figs. 14 and 15 illustrate a lock mechanism for connecting therapeutic wires or part thereof according to the present invention.
Figs. 16-32 illustrate the steps performed for positioning a coupling as shown in Fig. 2.
Fig. 33 is a top plan view of a distal part of a third embodiment of a second catheter having two lumens and being designed as an intravenous catheter according to the invention for use in a method of bypassing, a blocking in an artery according to the present invention.
Fig. 34 is a side-sectional view of the third embodiment of the second catheter shown in Fig. 33.
Figs. 35-47 illustrate the steps performed for positioning a coupling as shown in Fig. 2 and using the intravenous catheter shown in Figs. 33-34.
Figs. 48-56 illustrate the steps of a further method using a catheter according to Figs. 12-13 for positioning a coupling as shown in Fig. 2.
Figs. 57 and 58 illustrate two embodiments of a capturer used for performing the coupling shown in Fig. 2.

### Description of preferred embodiments

As stated above, it is an anatomical peculiarity that most of the arteries in the human body are in a very close proximity with a vein draining blood from the same area as the artery supports. Also, the veins are usually much bigger in diameter than the arteries, an observation that is of great importance for the present invention, since the catheter system according to the present invention is adapted to use such a wide vein as a medium to navigate in for the proper placement of a covered stent and also lends some of its space (diameter) for passing a new conduit past the block in the adjacent artery without blocking the flow in the vein itself.

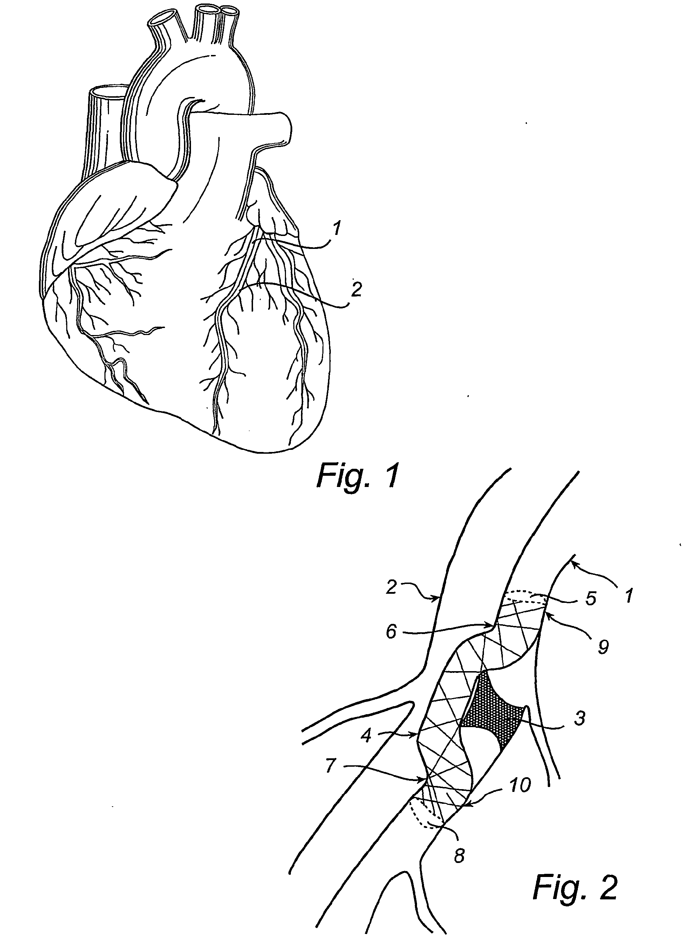

Fig. 1 shows the anatomy of the heart seen from the front and shows the close relationship between arteries 1 and veins 2. This close relationship is valid also between the veins and the arteries on the backside of the heart and further at many peripheral positions in the body.
Fig.2 illustrates a finished coupling bypassing a blocking 3 in an artery 1 of the heart. This coupling comprises a covered stent 4, which extends from a first point 5 within the artery upstream of the blocking 3, via a connection 6 out from the artery 1 into an adjacent vein 2, within the vein 2 past the blocking 3 in the adjacent artery 1, via a connection 7 out from the vein 2 and back into the adjacent artery 1, and to a second point 8 within the artery 1 downstreams of the blocking 3. A proximal end 9 of the covered stent 4 is fixed in the artery 1 at least at the first point 5, and a distal end 10 of the covered stent 4 is fixed in the artery 1 at least at the second point 8. The cross-section area of the covered stent 4 is smaller than the cross-section area of the vein 2, whereby the vein 2 still is able to let a stream of blood flow back towards the right atrium past the blocking 3 in the adjacent artery 1. At the same time, a stream of blood is able to pass from the heart through the artery 1 to the covered stent 4 upstream of the blocking 3 and via the covered stent 4 to the artery 1 downstream of the blocking 3, and then further out into the ramification of the artery 1.

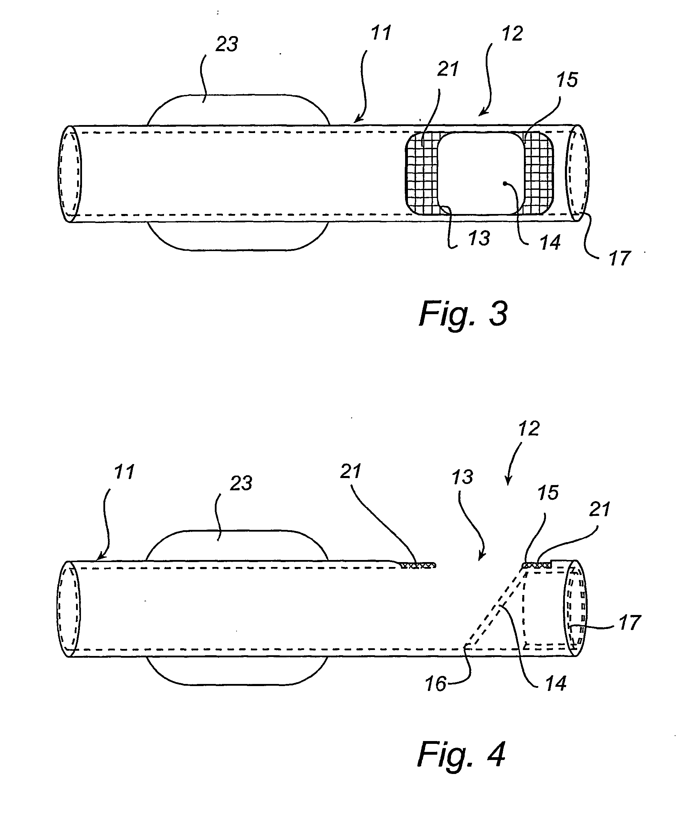

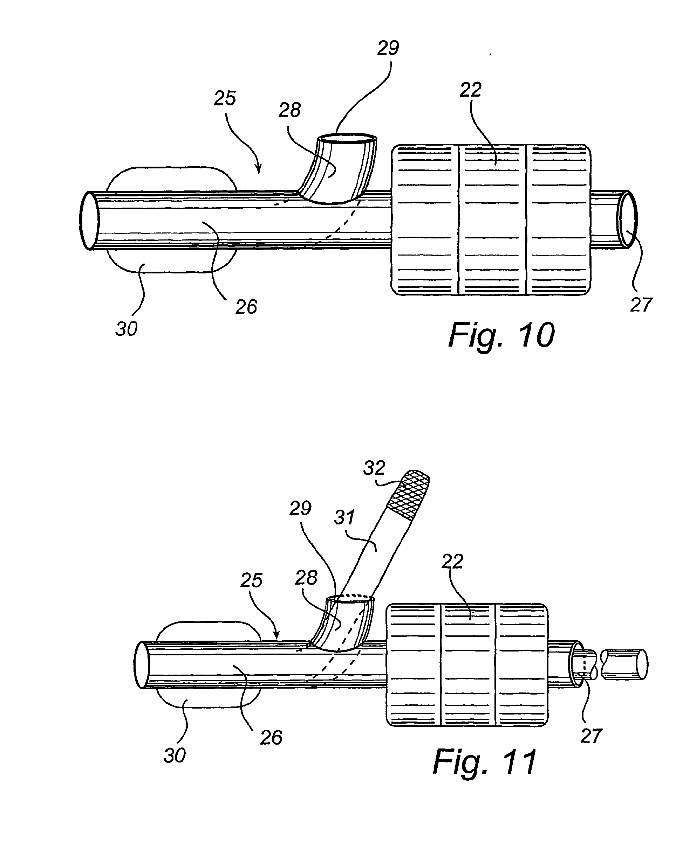

Figs. 3-15 illustrate preferred embodiments of an instrumental system that may be used to perform the method, whereby the finished connection illustrated in Fig. 2 may be obtained. The most essential parts of this system are an arterial catheter and an intravenous catheter, illustrated in Figs. 3-13.

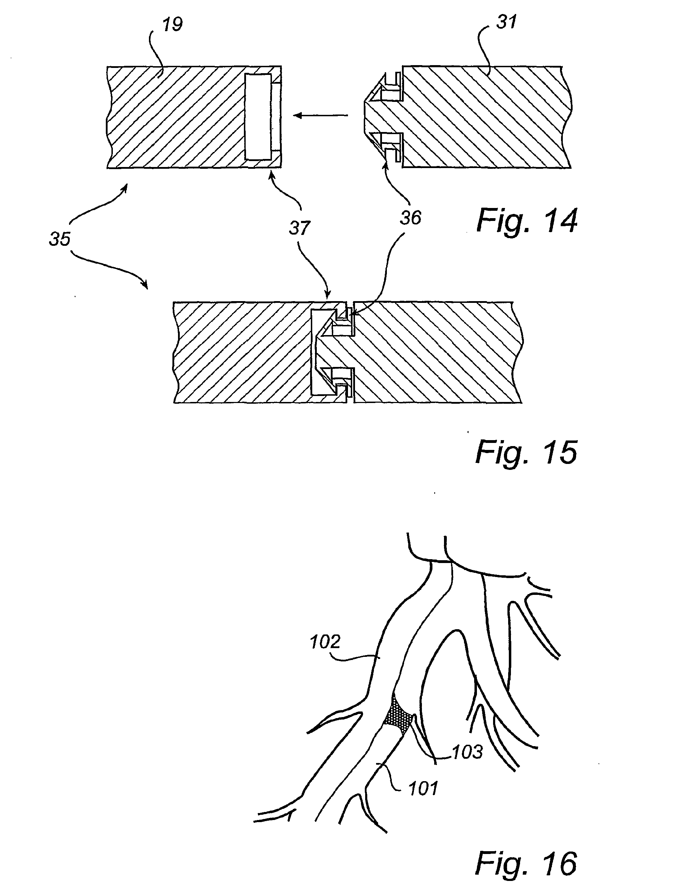

The preferred embodiment of the instrumental system of the present invention further comprises a wire system including conventional guide-wires and therapeutic wires having lock mechanisms illustrated in Figs. 14 and 15.

Figs. 3-7 illustrate a first embodiment of an arterial catheter 11, which at a distal end 12 has a lateral opening 13. A flap or tongue 14 is connected to the catheter 11 at a distal edge 15 of the lateral opening 13. This flap 14 is inclined towards the opposite side of the arterial catheter 11 and has a free end 16 proximal to the distal edge 15.

Consequently, a wire being guided through the arterial catheter towards the distal end 12 will be deflected by the flap 14 to project out through the lateral opening 13.

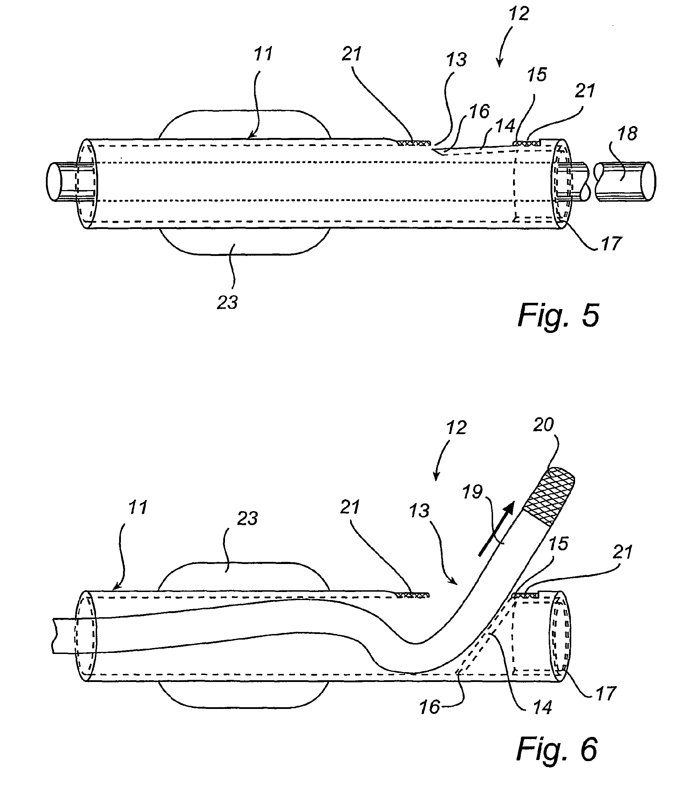

The arterial catheter 11 also has an opening 17 axially at its distal end, and the flap 14 preferably is flexible to a position substantially covering the lateral opening 13. Thereby, a wire 18 may be introduced into the arterial catheter 11, or vice versa, via the opening 17 and past the flap 14, which then will deflect towards the lateral opening 13, as shown in Fig. 5.

When using the arterial catheter 11 it is inserted into an artery, e.g. the artery 1 in Fig. 2, such that the lateral opening 13 is positioned opposite to the adjacent vein 2 directly upstream of the block 3. Then a wire 19 having a cutting tip 20 is inserted through the arterial catheter 11 such that the cutting tip 20 will be deflected laterally by the flap 14 out of the lateral opening 13, through the wall of the artery 1 and the wall of the adjacent vein 2 into this vein 2. The cutting or active end or tip 20 is capable of cutting through the wall of a blood vessel and may use radiofrequency energy, laser energy, a sharp end, or other cutting techniques known in the art.

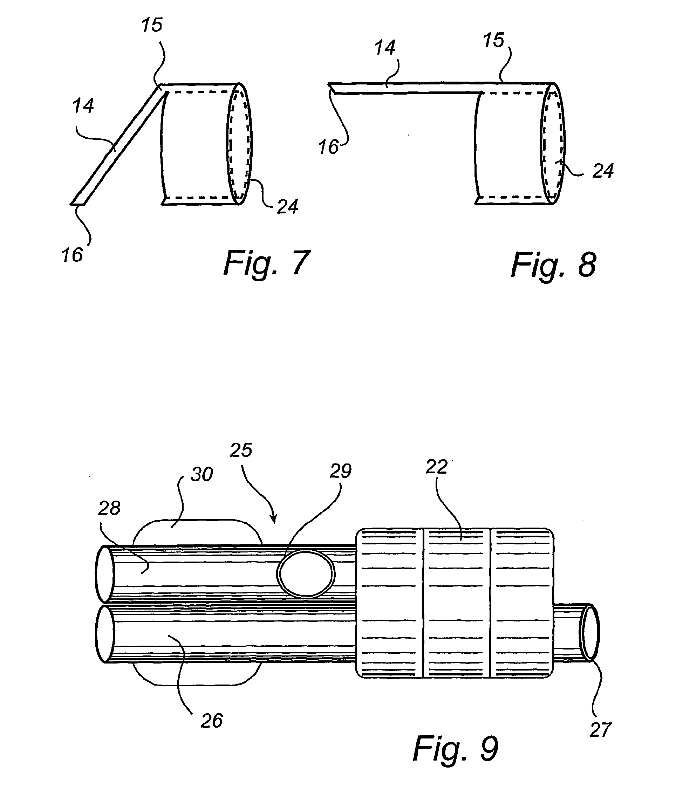

In order to be able to perform this operation, it must be possible to determine the rotational position of the lateral opening 13 at the distal end 12 of the arterial catheter 11 within an artery into which the arterial catheter 11 is inserted. Preferably, this is attained by attaching a rotational position indicator 21 to the distal end of the arterial catheter and introducing a detector 22 (cf. Figs. 9-13) through the adjacent vein to a position close to the rotational position indicator 21, this detector 22 being sensitive to the rotational position of the rotational position indicator 21.

Further, a balloon 23 is provided at the distal part 12 of the artery catheter 11. The balloon 23 is inflated when the rotational position of the distal opening 13 of the artery catheter 11 is opposite to the adjacent vein, as determined by the rotational position detector 22. Thereby, the position of the artery catheter 11 is fixed relative to the adjacent vein.

As a preferred example, the position indicator 21 may be an ultrasound reflecting material provided non-concentric on the arterial catheter 11 near the distal opening 13, e.g. so as to at least cover part of the edge around the distal opening 13. In this case, the detector 22 should comprise an ultrasound head, which is able to detect the rotational position of the arterial catheter 11.

Other types of indicators and detectors, such as a magnetic indicator and a magnetic detector, are of course possible. Each type of indicator and detector may be used separately or in combination with another type of indicator or detector.

It is also possible that the material of the arterial catheter 11 is such as to enable a detecting of the rotational position of the distal end 12 thereof as a consequence of the presence of the opening 13 and/or the flap 14 which themselves are non-concentric in relation to a central axis of the arterial catheter 11. Thus, this would eliminate any extra material being applied to the distal end 12 of the arterial catheter 11 to make it indicative of its rotational position.

Figs.7 and 8 illustrate an exemplary design of the flap 14. More precisely, the flap 14 is fixed to a ring 24 which may be inserted into the opening 17 of the catheter 11 and fixed therein adjacent to the opening 13, as illustrated in Figs. 4-6. Fig. 7 shows the flap 14 in its inclined position, and Fig. 8 the flap 14 in its upward towards the opening 13 flexed position. Other embodiments of the flap 14, e.g. a flap integrated in the catheter, are obvious to the man skilled in the art.

A first embodiment of the intravenous ultrasound therapeutic catheter 25 is illustrated in Figs. 9-11, This catheter 25 is a typical angiography catheter of the double lumen type wherein a first lumen 26 has an axially directed distal opening 27 and a second lumen 28 has a substantially laterally directed distal opening 29. The catheter 25 further has an ultrasound head constituting said detector 22 and being directed substantially in the same lateral direction as the distal opening 29 of the second lumen 28. Finally, the catheter 25 has a balloon 30 whereby its rotational and axial position with a vein may be fixed. As illustrated in Figs. 9-11, the detector 22 is disposed distally of the opening 29. However, the detector 22 may be disposed proximally of the opening 29, in which case the balloon 30 preferably is disposed distally of the opening 29.

The intravenous ultrasound therapeutic catheter 25 first is used for making a connection upstream of the blocking from the arterial catheter 11 positioned in an artery into the vein in which the catheter 25 is introduced. Thus, the ultrasound head or detector 22 of the catheter 25 is used for determining the rotational position of the distal end 12 of the arterial catheter 11. When it is determined that the opening 13 is directly opposite to the vein, this rotational position of the distal end 12 of the arterial catheter 11 is fixed by inflating the balloon 23. Then, the wire 19 having the cutting tip 20 is inserted through the arterial catheter 11 such that the cutting tip 20 will be deflected laterally by the flap 14 out of the lateral opening 13, through the wall the artery and through the wall of the adjacent vein into this vein.

The intravenous ultrasound therapeutic catheter 25 thereafter is used for making a connection downstream of the blocking from the vein in which the catheter 25 is positioned to the same artery from which a connection first was made. Now, the ultrasound head 22 is used to set the rotational position of the catheter 25 such that the opening 29 of the second lumen 28 of the catheter 25 is directly opposite to and thus points toward the artery. The balloon 30 of the catheter 25 is now inflated in order to fix this rotational position of the catheter 25 in the vein. Then, a wire 31 having a cutting tip 32 is inserted through the second lumen 28 of the catheter 25 such that the cutting tip 32 will be deflected laterally out of the distal opening 29 of the second lumen 28, through the wall of the vein and through the wall of the adjacent artery into this artery. The wire 31 is of the same type as the wire 19.

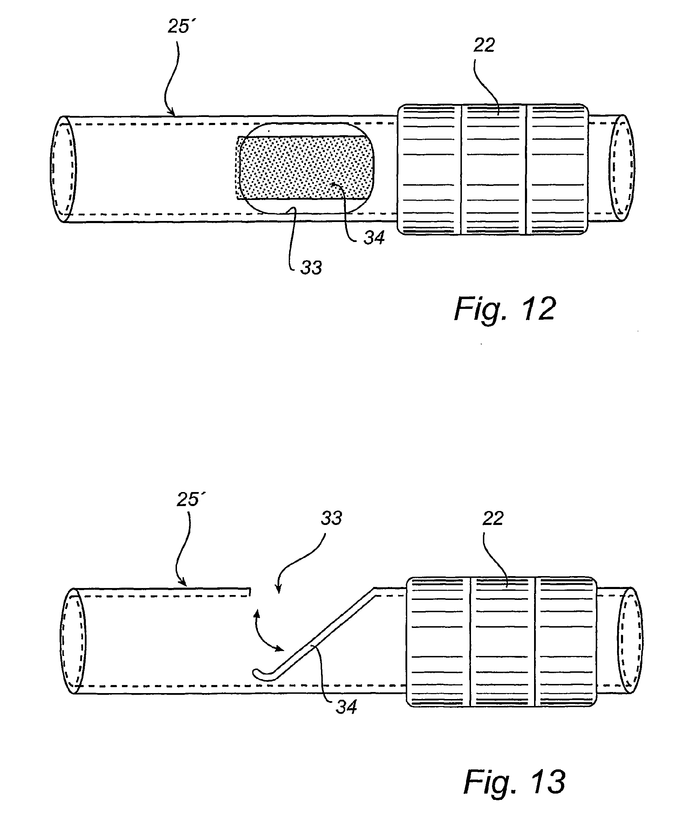

A second embodiment of the intravenous catheter is illustrated in Figs.12 and 13. This intravenous ultrasound therapeutic catheter 25' has the same ultrasound detector 22 as the catheter 25 but is a single lumen catheter, which has a lateral opening and a flap 34. The lateral opening 33 and the flap 34 corresponds to the lateral opening 13 and the flap 14 of the first, arterial catheter 11.

When using the intravenous catheter 25', a guide-wire is first introduced into the vein and the catheter 25' is then pushed on this guide-wire to the area of the blocking in the artery. During this movement of the catheter 25', the flap 34 is flexed into a position substantially corresponding to the lateral opening 33.

In order to make the connection from the vein into the artery downstream of the blocking, the guide-wire is retracted from the catheter 25' and an active therapeutic wire, i.e. a therapeutic wire having an active tip or end, is introduced from the proximal of the catheter 25'. When the cutting head of the active wire reaches the flap 34 it is deflected through the lateral opening 33, whereby a connection from the vein and into the artery will be made.

Since the ultrasound transducer 22 of both catheters 25 and 25' is monitoring a sector the openings 29 and 33, respectively, the movement of the therapeutic wire 31 out of these openings is easily controlled.

Further elements of the instrumentation system according to the present invention are in Figs.14 and 15, which illustrate a lock mechanism 35 for connecting the ends of two wires, such as the wires 19 and 31 or an active head to the end of a wire, such the head 20 to the wire 19. The lock mechanism 35 comprises a flexible male member 36 and a female member 37, which are easily connected and disconnected. Of course, modifications of this lock mechanism are obvious to the man skilled in the art. It should be noted that the lock mechanism 35 does not increase the thickness of the wires 19 and 31 and that the flexibility thereof is hardly affected at all.

Having described embodiments of the arterial catheter and the intravenous catheter as well as their basic functions, a complete description of a method of bypassing a blocking will now be made referring to an example illustrated in Figs. 16-32.

An artery 101 and a parallel vein 102 of a heart are illustrated in Fig. 16. Also, an occlusion or blocking 103 is shown in the artery 101.

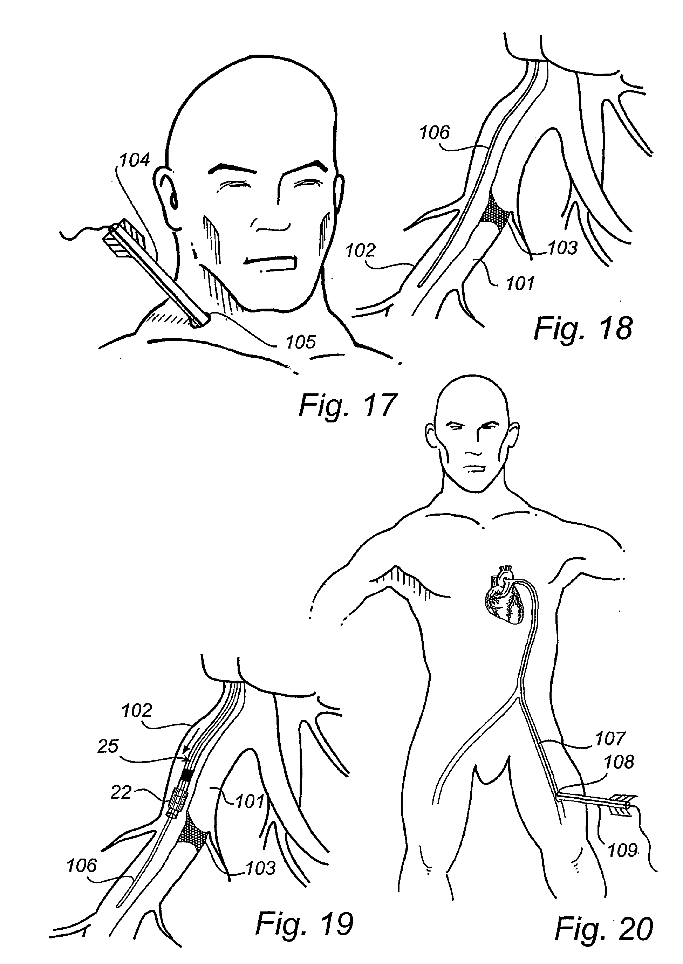

Via a catheter sheet 104 introduced at a puncture site 105 of the jugular vein, as illustrated in Fig. 17, an ordinary guide-wire 106 is inserted into the vein 102 past the position of the occlusion in the artery 101, cf. Fig. 18. An intravenous ultrasound therapeutic catheter, such as the catheter 25, is then inserted over this guide-wire 106, cf. Fig. 19. The active ultrasound head 22 of the catheter 25 is now used to localise the block 103 in the artery 101.

The information collected by means of the ultrasound head 22 is also used to determine whether the anatomical conditions are good enough for making a coupling according to the present invention.

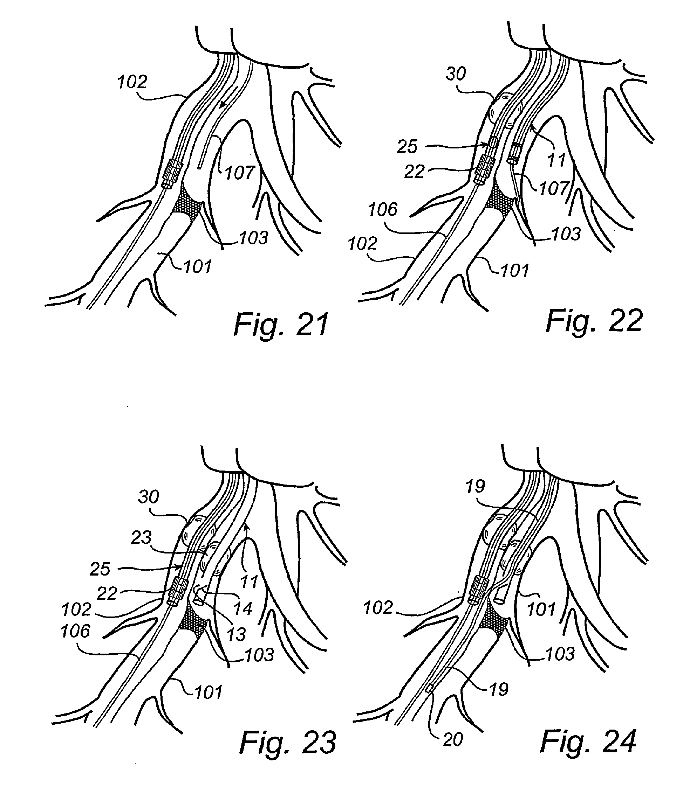

Presuming that acceptable conditions are confirmed, a second common guide-wire 107 is now introduced at a puncture site 108 from the femoral artery in the groin (or any other peripheral artery) using a catheter sheet 109 and advanced all the way to the block 103 in the coronary artery 101, cf. Figs. 20 and 21.

The balloon 30 of the catheter 25 is inflated so as to lock the catheter 25 in a position opposite to the artery 101 and proximal to the block 103. An arterial catheter, such as the catheter 11, is then inserted over this guide-wire 107, cf. Fig. 22.

Using the active ultrasound head 22 of the catheter 25 in the vein 102, the reflecting material around the opening 13 at the distal end of the arterial catheter 11 is detected, whereby it is possible to rotate the arterial catheter 11 until its opening 13 is facing directly towards the vein 102. In this position the balloon 23 of the catheter 11 is inflated so as to fix the rotational and axial position of the distal part 12 of the arterial catheter 11 in the coronary artery 101. As a next step, the guide-wire 107 is retracted whereby the flap 14 returns to its inclined position, cf. Fig. 23.

Then, the wire 19 having the cutting tip 20 is inserted through the arterial catheter 11 such that the cutting tip 20 will be deflected laterally by the flap 14 out of the lateral opening 13, and will make a connection through the wall of the artery 101 and through the wall of the adjacent vein 102 into this vein 102, cf. Fig. 24. This movement of the wire 19 and its tip 20 may be monitored by means of the ultrasound head 22 of the catheter 25 in the vein 102. Eventually, the wire 19 extends proximal to the block 103 in the artery 101, via the connection made into the vein 102 and to a position in the vein 102 that is distal to the block 103 in the coronary artery 101.

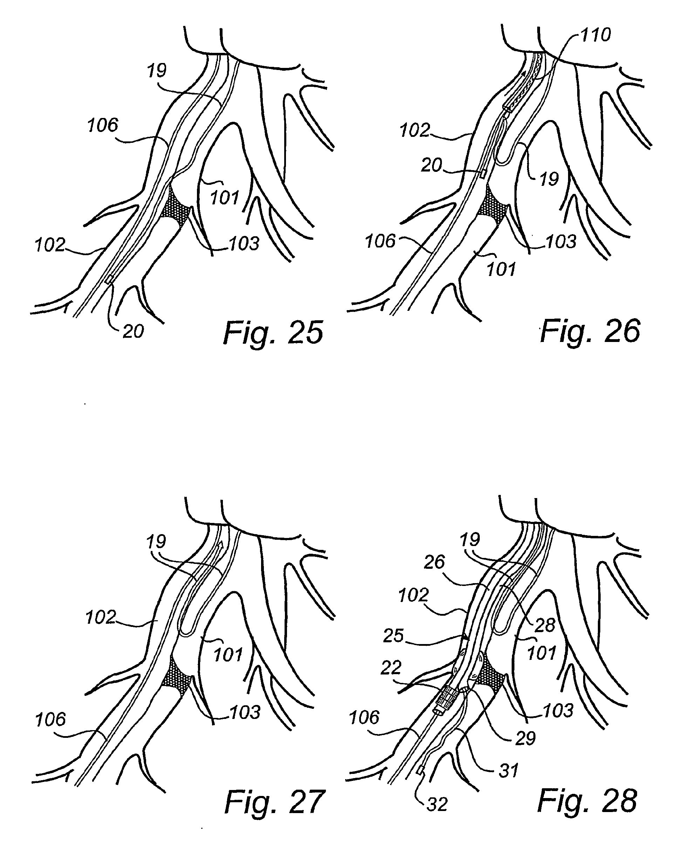

The balloons 23 and 30 may now be deflated and the catheters 11 and 25 withdrawn from the coronary artery 101 and the coronary vein 102, respectively. This is depicted in Fig. 25.

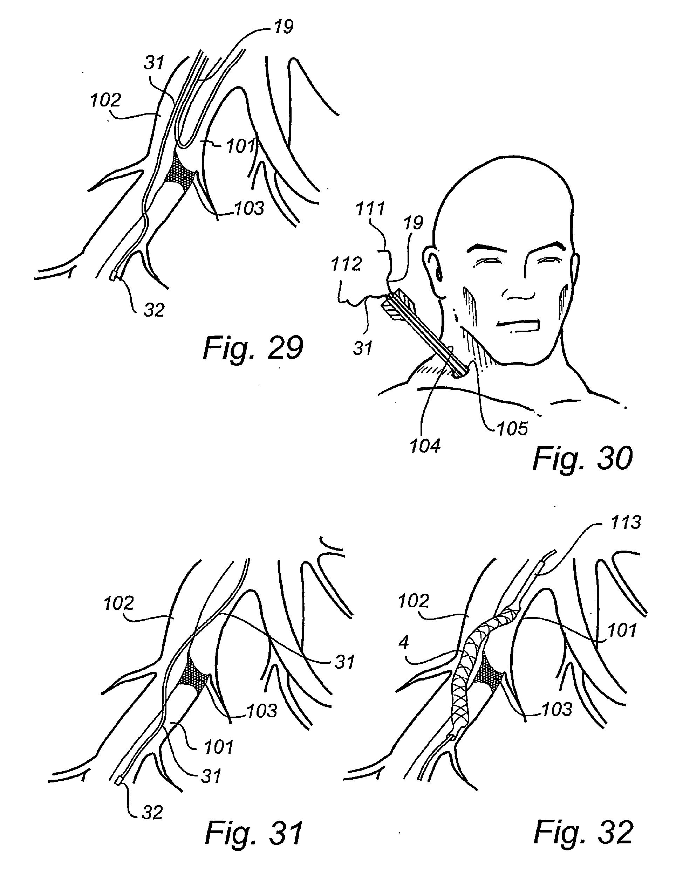

Thereafter, another catheter 110 is introduced over the guide-wire 106 to the area of the block 103, where the distal part of the wire 19 is caught and then retracted out of the vein 102 together with the catheter 110, cf. Fig. 26. Thus, the wire 19 now extends from puncture site 108 of the femoral artery in the groin to the block 103 in the coronary artery 101, via the connection through the walls of the coronary artery 101 and the coronary vein 102 into the vein 102 and then back to and out of the puncture site 105 of the jugular vein, cf. Figs. 27 and 30.

Next, the intravenous ultrasound treatment catheter 25 is again introduced at the puncture site 105 of the jugular vein and is inserted on the guide-wire 106 in parallel with the wire 19 to the area of the block 103. After localising the distal part of artery 101 by means of the active ultrasound head 22, the balloon 30 will be inflated and the position of the catheter 25 thereby fixed. The wire 31 having the cutting head 32 is introduced through the second lumen 28 of the catheter 25. Once it reaches the end of the second lumen 28 it will be deflected out of the lateral opening 29 towards the coronary artery 101 and make a connection through the wall of the coronary vein 102 and the wall of the artery wall 101 into the artery 101, cf. Fig. 28.

Now, the intravenous ultrasound treatment catheter 25 is retracted from the coronary vein 102, cf. Fig. 29, and the ends 111 and 112 of the wires 19 and 31 outside the puncture site 105 are connected so that they effectively form a single wire 19, 31, cf. Fig. 30. As a result, the wire 19 may be withdrawn from the femoral artery in the groin to such an extent that said single wire 19, 31 extends from the femoral artery in the groin to a point proximally of the block 103 in the coronary artery 101, via the connection through the walls of the coronary artery 101 and the coronary vein 102 into the coronary vein 102, through a part of the coronary vein 102 past the block 103 in the coronary artery 101, via the connection through the walls of the coronary vein 102 and the coronary artery 101 back into the artery 102 distally of the block 103, cf. Fig. 31.

Finally, a covered stent 4, e.g. as described in U.S. Patent 6,395,212 (published late) (published late) (JAN OTTO SOLEM), or any other covered stent graft is mounted on a balloon on a catheter 113 and is inserted over the single wire 19, 31 from the femoral artery through the coronary artery 101, through the connection in the walls of the coronary artery 101 and the coronary vein 102 proximally of the block 103, through part of the coronary vein 102, through the connection in the walls of the coronary vein 102 and the coronary artery 101 distally of the block 103, back into the coronary artery distally of the block 103. Now, the proximal end of the covered stent is positioned in the coronary artery 101 proximal to the blocking 103, and a distal end of the covered stent is positioned in the artery 101 distal to the blocking 103, cf. Fig. 32.

Finally, the proximal and distal ends 9, 10 of the covered stent 4 are fixed in the coronary artery 101 by inflating said balloon, which is then deflated and withdrawn with the catheter 113. Thereby, the covered stent 4 will bypass the block 103 in the coronary artery 101, as also shown in Fig. 2. It should be noted that since the vein 102 is much wider than the covered stent, i.e. the cross-sectional area of the vein is substantially larger than that of the covered stent (and the coronary artery), the bypass does not obstruct the flow of blood in the coronary vein.

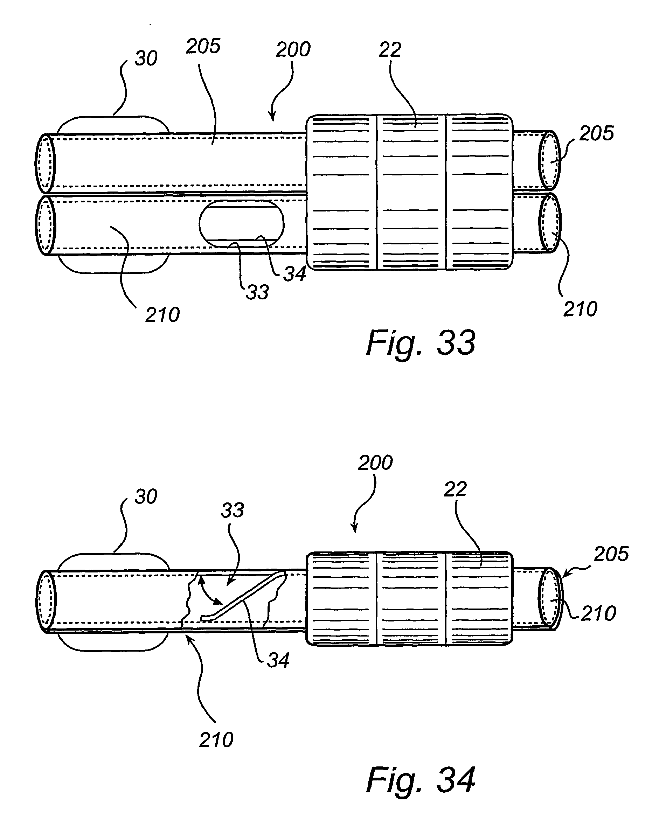

Referring to Figs. 33-47, an apparatus will be described which represent an alternative to those described referring to Figs. 16-32. Here a single catheter is used in the coronary vein 102 for both capture of the wire 19 and for advancement of the wire 31 from the vein 102 into the coronary artery 101, thereby reducing the number of exchanges and the amount of apparatus required.

As seen in FIG. 33, a catheter 200 comprises a capture lumen 205, a guide wire lumen 210, a position detector 22 and a balloon 30. The position detector 22 may provide a position indication with ultrasound, MRI, OCT, angiography, radiography, or any other position indication technique known in the art. The capture lumen 205 has an axially directed distal opening. The guide wire lumen 210 also has an axially directed distal opening and further comprises a lateral opening 33 a flap 34, as described above. FIG. 34 is a side-sectional view of the catheter 200, illustrating the flap 34 in its inclined configuration.

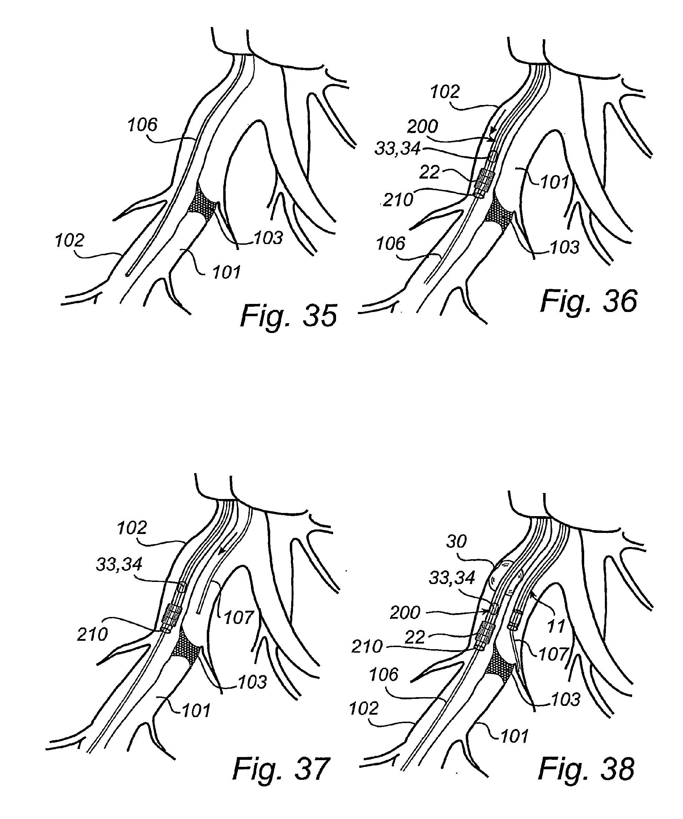

As seen in FIG. 35, the guide wire 106 is advanced through the patient's jugular vein into the patient's coronary vein 102 using the technique described with respect to FIGS. 17 and 18. The distal end of the guide wire lumen 210 of the catheter 200 is then advanced over the proximal end of the guide wire 106, thereby flexing the flap 34 towards the lateral opening 33 to allow advancement of the catheter 200 into the coronary vein 102, as seen in Fig. 36. Figs. 37-40 then correspond to Figs. 21-24.

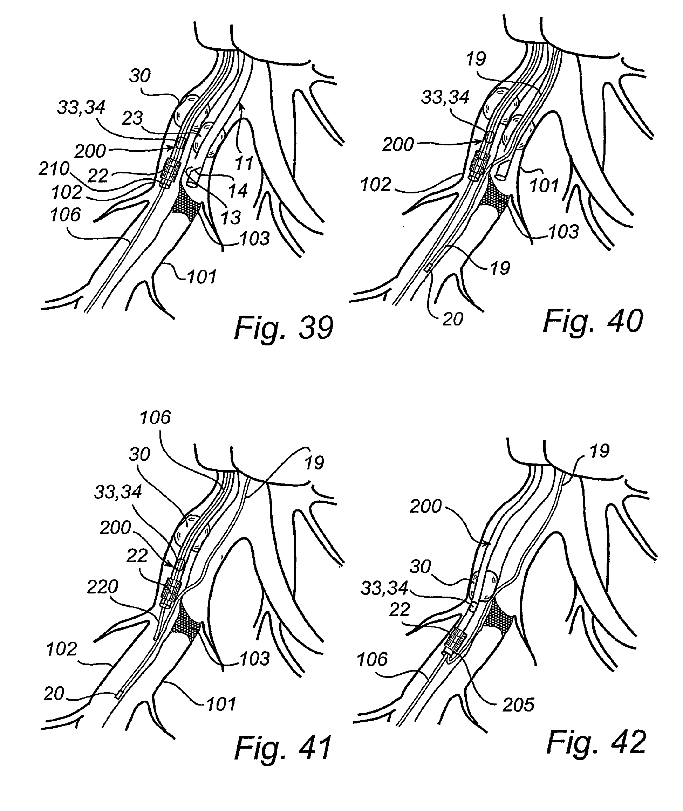

Referring now to Fig. 41, the guide wire 106 is withdrawn proximally a distance sufficient to make way for introduction of a capture device 220. Preferably, the guide wire 106 is retracted to a position proximal of the distal end of the guide wire lumen 21 but distal to the flap 34. The capturer 220, or any other suitable capture device, is then advanced through the capture lumen 205 and out of its distal end. The capturer 220 captures the distal region of the wire 19. Capture of the wire 19 optionally may be facilitated by an imaging modality, for example IVUS, OCT, MRI, angiography, fluoroscopy, etc.

In Fig. 42, the wire 19 is then withdrawn through the capture lumen 205 such that it from the femoral artery, through the coronary artery 101, into the coronary vein 102, and but of the jugular vein. The guide wire 106 is then re-advanced distally to a position distal of the block 103. The balloon 30 is deflated, and the catheter 200 is advanced further along wire 106 until the lateral opening 33 is disposed a position distal of the block 103. The lateral opening 33 is then aligned with the coronary artery 101 using, for example, one of the techniques described previously. The balloon 30 is then re-inflated to maintain the orientation and position of the opening 33.

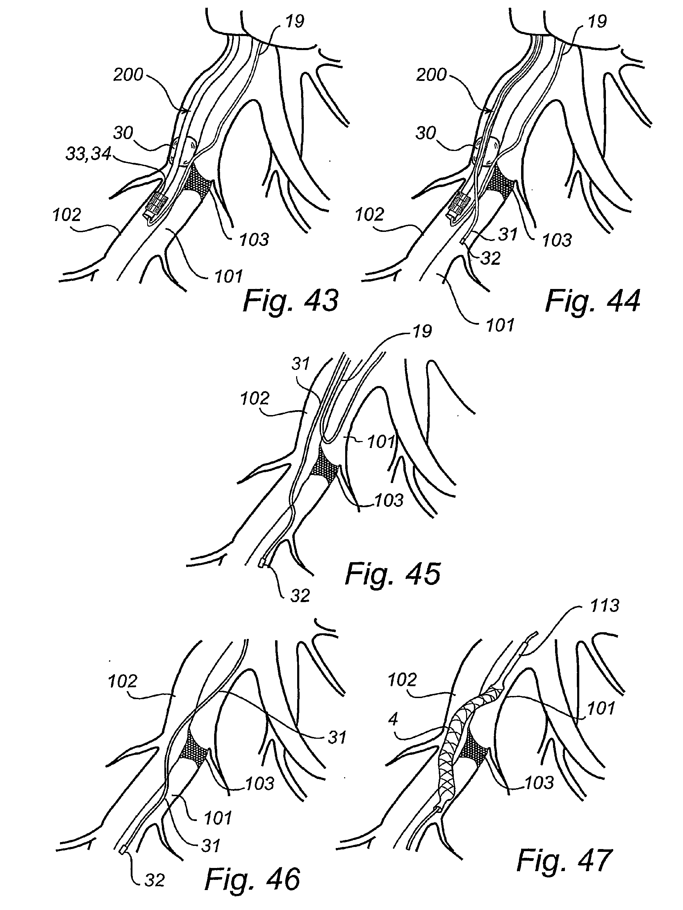

In Fig. 43, the guide wire 106 is withdrawn through the guide wire lumen 210, thereby opening the lateral opening 33. Then, as seen in Fig. 44, the active wire 31 having a cutting tip 32 is advanced through the lumen 210 and is deflected by the flap 34 out of the lateral opening 33. Continued advancement of the wire 31 causes the cutting tip 32 to puncture the wall of the vein 102 and to enter the artery 101 distal of the block 103. The balloon 30 is then deflated, and the catheter 200 is removed from the vein 102. The proximal end of the wire 31 is then connected to the distal end of the wire 19, and Figs. 45-47 then correspond to Figs. 29 31 and 32.

It should be understood that the guide wire 106 and/or the guide wire 107 could be eliminated from the procedure, and the active wires 19 and 31 used in their place. This may simplify the procedure by reducing the number of exchanges required, i.e. by not having to completely remove the guide wires, then advance the active wires. It is expected that such a system would be most effective if used in combination with active wires having cutting tips that may be selectively activated, for example, by a laser or RF-source.

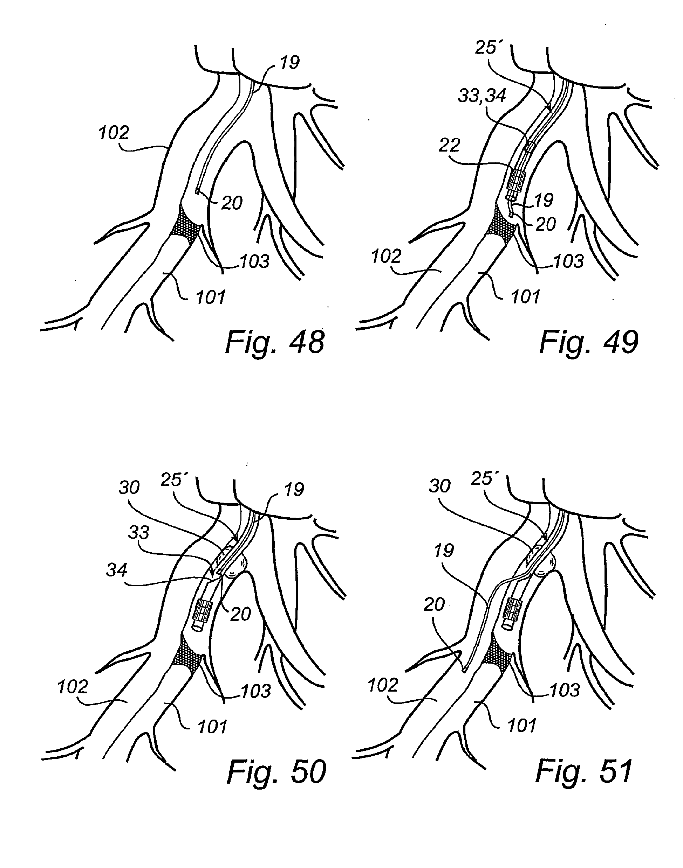

Referring to Figs. 48-56 an apparatus and a method are disclosed which utilise the apparatus 25' described in Figs. 12 and 13 in conjunction with the balloon 30.

In Fig. 48, the wire 19 having a cutting tip 20 is introduced into the coronary artery 101, using well-known percutaneous techniques, to a position just proximal of the block 103. The distal end of the catheter 25' is advanced over the proximal end of the wire 19, such that the flap 34 deflects towards the lateral opening 33 and allows advancement of the catheter 25' to a position just proximal of the block 103, as seen in Fig. 49. The catheter 25' may then be rotated such that the lateral opening 33 is aligned with the coronary vein 102. Proper alignment may be achieved using a variety of techniques. For example, the angular position of the opening 33 with respect to the ultrasonic detector 22 may be tagged on an intravascular ultrasound ("IVUS") image derived therefrom (not shown). Then, fluoroscopic, ultrasound, angiographic, MRI, OCT, etc., images of the coronary vein 102 and/or coronary artery 101 may be compared with the IVUS image to orient the tag such that the lateral opening 33 is directed towards the vein 102. Alternatively, the position indicator and detector system described previously may be used.

Once the lateral opening 33 is properly aligned, the balloon 30 is inflated to maintain alignment. As seen in Fig. 50, the wire 19 is then withdrawn proximally to a position proximal of the flap 34, thereby causing the flap 34 to return to its inclined position and re-opening the lateral opening 33. It should be understood that a standard guide wire, such as the guide wire 106, may alternatively be used to position the catheter 25' within the vessel, then withdrawn and replaced by the wire 19 having the cutting tip 20.

With the lateral opening 33 re-opened, the wire 19 is re-advanced distally such that it is deflected by the flap 34 to project out of the lateral opening 33. Continued advancement of the wire 19 causes the cutting tip 20 to puncture the wall of the coronary artery 101 and to pass into the coronary vein 102. The wire 19 is then advanced until its distal end is disposed distal of the coronary block 103, as seen in Fig. 51.

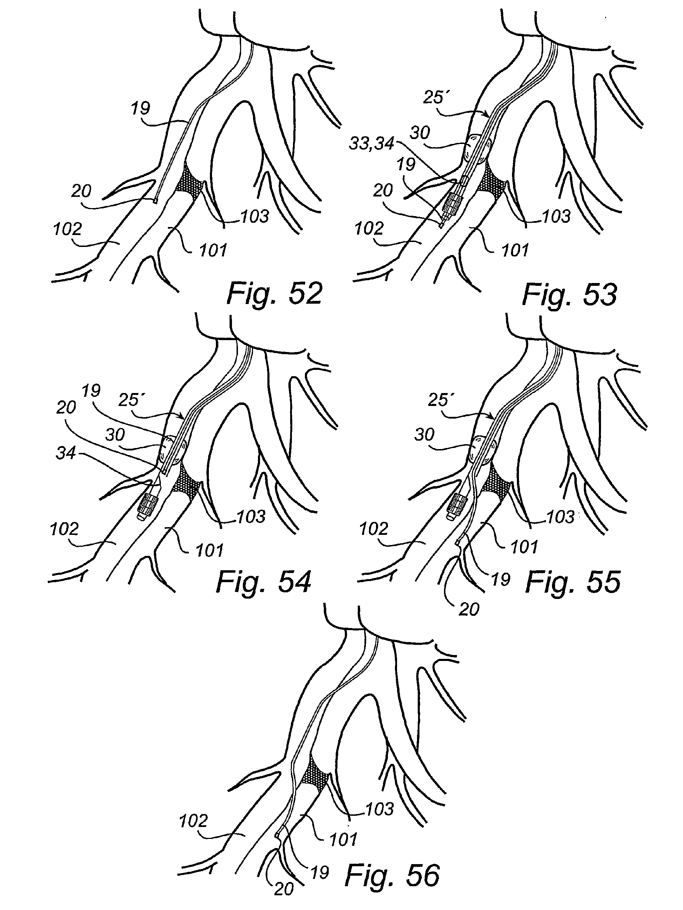

Once the distal end of the wire 19 is positioned within the patient's coronary vein 102, the balloon 30 is deflated, and the catheter 25' is removed from the patient's vasculature, as seen in Fig. 52. The distal end of the catheter 25' is preferably flexible to reduce damage to the wall of the artery 101 during removal. Once removed from the patient, the distal of the catheter 25' is re-advanced over the proximal of the wire 119 thereby re-closing the lateral opening 33 by deflecting the flap 34. Advancement of the catheter 25' continues until the distal end of the catheter 25' is disposed within the patient's coronary vein 102 distal of the block 103. Lateral opening 33 is then aligned with the coronary artery 101 using, for example, one of the techniques described hereinabove, or by imaging block 103. The balloon 30 is re-inflated to maintain the orientation of the opening 33, as seen in Fig. 53.

Referring to Fig. 54, the wire 19 is withdrawn proximally until it is disposed proximal of the flap 34, thereby causing the flap 34 to deflect downward and opening the lateral opening 33. The wire 19 is re-advanced distally such that it is deflected by the flap 34 to project out of the lateral opening 33. Continued advancement causes the cutting tip 20 to puncture the wall of the coronary vein 102 and to pass back into the coronary artery 101, as seen in Fig. 55. The balloon 30 is then deflated, and the catheter 25' is withdrawn from the coronary vein 102 through the coronary artery 101, and out of the patient's vasculature, as seen in Fig. 56. The flexibility of the distal region of the catheter 25' again serves to minimize trauma to the vasculature during retrieval of the catheter.

The wire 19 thus extends from a point external to the patient, through the patient's vasculature to a point just proximal of the block 103 in the coronary artery 101, into the coronary vein 102 via the connection through the walls of the artery 101 and the vein 102, through a part of the coronary vein to a position past the block 103, and back into the artery 101 distal of the block 103 via the connection between the vein 102 back into the artery 101. The covered stent 4 is then advanced over the wire 19 and deployed as described with respect to Fig. 32.

Referring back to Figs. 53-56, it should be understood that the catheter 25' may alternatively be re-advanced along the wire 19 using well-known rapid exchange techniques (not shown), or via a hypotube or additional lumen provided as part of catheter 25' (not shown). In such a system, the catheter 25' would be re-advanced with the lateral opening 33 in the open configuration. A second active wire with a cutting tip, such as the wire 31 having the cutting tip 32, would then be advanced through the catheter 25' and out of the opening 33 to make the return connection between the coronary vein 102 and the coronary artery 101 distal of the block 103. The catheter 25' would then be removed from the patient, followed by the wire 19, leaving the wire 31 in position for placement of the covered stent 4. It is expected that such a system may reduce trauma at the arterio-venous connection proximal of the block 103 during retrieval of the catheter 25' due to its guidance along the wire 19 during retrieval, which would extend parallel to the longitudinal axis of the catheter 25' rather than through the lateral opening 33.

The cutting tip 20 of the wire 19 is preferably selectively activated to minimize a risk of perforating the patient's vasculature at locations other than the arterio-venous connections seen, for example, in Fig. 56. The cutting tip 20 may, for example, be coupled via the wire 19 to a selectively activated radiofrequency or laser energy source.

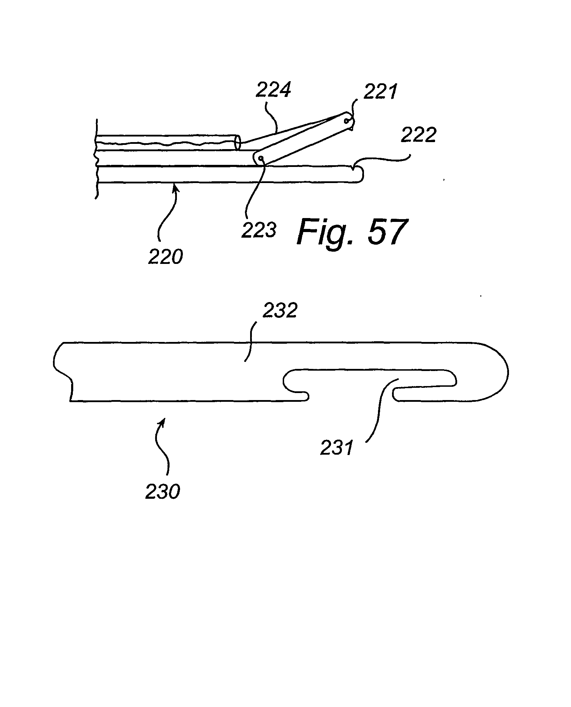

Fig. 57 provides an illustrative capturer 220 suited for advancement through capture lumen 210. The capturer 220 comprises two jaws 221, 222 being urged together by a spring 223. Pulling a thread 224 may open the jaws 221, 222.

Fig. 58 illustrates another capturer 230, which comprises a hook 231 formed in the side of metal string or wire 232.

Alternative capture devices known from the art may also be provided, for example an Amplatz Gooseneck snare, distributed by the Microvena Corp. of White Bear Lake, Minn., USA.

It should be noted that several modifications might be made to the above-described example of the invention and the method. As an example, the catheter 25' may be used instead of the catheter 25. Also, the intravenous catheter 25 or 25' may be used in the artery 1 to make the first connection 6 and thereafter be retracted from the artery and used in the vein 2 as above described. Alternatively, both catheters may be equipped with position detectors providing a position indication with ultrasound, MRI, OCT, angiography, radiography, or any other position indication technique known in the art.

Further, magnets or electromagnets may be used for indication of position. Thus, if a magnet or electromagnet was disposed on the catheter in the vein and another magnet or electromagnet was disposed on the catheter in the artery, they could lock on to each other when in proper position. The magnets could be disposed only on the sides of the catheters with lateral openings to ensure proper alignment. Additionally, magnets or electromagnets could eliminate the need for a balloon for anchoring during creation of the first and second connections.

## Claims

1. A catheter system comprising:
a guiding catheter (11) for introduction into a human body vessel, said catheter (11) comprising:
a distal lateral opening (13), and
a longitudinal guiding lumen,
**characterised in that** said catheter (11) during said introduction has a first configuration in which said lateral opening is essentially blocked and
wherein said catheter (11) when positioned in said body vessel has a second configuration in which said lateral opening is unblocked and said lumen ends in said lateral opening, a transfer from the first to the second configuration, being controllable by a longitudinal movement of a wire extending through said longitudinal lumen.

2. The catheter system according to claim 1, wherein said guiding catheter (11) further comprises a distal axial opening and wherein, in said first configuration of the catheter (11), said longitudinal guiding lumen ends in said axial opening.

3. The catheter system according to claim 1 or 2, wherein said guiding catheter (11) further comprises a guide means (14), which essentially blocks said lateral opening (13) in the first configuration of the catheter (11) and which is arranged to guide a wire through said lateral opening (13) in the second configuration of the catheter (11).

4. The catheter system according to any one of the preceding claims, further comprising a cutting wire (19) having a cutting tip (20) at a distal end thereof, said cutting wire (19) being advancable through the guiding catheter (11) so as to project its cutting tip (20) through the unblocked lateral opening (13) of the guiding catheter (11).

5. The catheter system according to claim 3 or 4, wherein the guide means comprises a flap (14), which is hingedly connected at the lateral opening (13) and which has a proximal free end.

6. The catheter system according to claim 5, wherein the flap (14) is moveable between a position essentially blocking the lateral opening (13) of the guiding catheter (11) and a position, wherein the flap (14) is inclined for guiding a wire through said lateral opening (13).

7. The catheter system according to any one of the preceding claims, further comprising a rotational position indicator (21) arranged on the guiding catheter (11).

8. The catheter system according to claim 7, wherein the rotational position indicator (21) comprises an ultrasound reflecting material at a predetermined position relative to the lateral opening (13) of the guiding catheter (11).

9. The catheter system according to claim 8, wherein the ultrasound reflecting material (21) at least partly encircles the lateral opening (13) of the guiding catheter (11).

10. The catheter system according to anyone of the preceding claims, further comprising an inflatable balloon (23) for fixing the position of the distal end (12) of the guiding catheter (11) in the body vessel.

11. The catheter system according to any one of claims 7-9, further comprising a positioning catheter (25; 25') for introduction into another human body vessel and having a detector (22) responsive to the rotational position indicator (21).

12. The catheter system according to claim 11, wherein the positioning catheter is an ultrasound catheter (25; 25') comprising an ultrasonic transducer (22) for monitoring a positioning of the lateral opening (13) of the guiding catheter (11) when introduced into said body vessel.

13. The catheter system according to claim 11 or 12, wherein the positioning catheter (25) comprises two parallel lumina (26, 28; 205, 210), one of which (26; 205) has an axially directed distal opening (27) and the other of which (28; 210) has a laterally directed opening (29; 33).

14. The catheter system according to claim 13, wherein the laterally directed opening (29) is a distal opening of the other one (28) of said parallel lumina.

15. The catheter system according to claim 14, further comprising a flap (34) connected to a distal edge of the laterally directed opening (29), said flap being inclined towards the opposite side of the other one (210) of said parallel lumina and having a free end proximal of said distal edge.

16. The catheter system according to claim 15, wherein the flap (34) is flexible to a position substantially covering the laterally directed opening (33) of the other one (210) of said parallel lumina.

17. The catheter system according to claim 16, wherein the other one (210) of said parallel lumina also has an axially directed distal opening.

18. The catheter system according to claim 12-17, wherein the ultrasonic transducer (22) is directed laterally substantially in the direction of the laterally directed opening (29) of the ultrasound catheter (25).

19. The catheter system according to claim 18, wherein the ultrasonic transducer (22) is positioned distal to the laterally directed opening (29).

20. The catheter system according to claim 11-19, further comprising an inflatable balloon (30) for fixing the position of the distal end of the positioning catheter (25; 25') in said other body vessel.

21. The catheter system according to any one of the preceding claims, wherein the guiding catheter (11) further comprises a second longitudinal lumen having an axially directed distal opening.

## Patentansprüche

1. Kathetersystem, das umfasst:
einen Führungskatheter (11) zur Einführung in ein menschliches Körpergefäß, wobei der Katheter (11) umfasst:
eine distale seitliche Öffnung (13), und
ein Längsführungslumen,
**dadurch gekennzeichnet, dass** der Katheter (11) während der Einführung eine erste Konfiguration besitzt, in welcher die seitliche Öffnung im wesentlichen blockiert ist und
wobei der Katheter (11), wenn er in dem Körpergefäß positioniert ist, eine zweite Konfiguration besitzt, in welcher die seitliche Öffnung unblockiert ist und das Lumen in der seitliche Öffnung endet, wobei ein Transfer von der ersten zu der zweiten Konfiguration durch eine Längsbewegung eines Drahtes, der sich durch das Längslumen erstreckt, steuerbar ist.

2. Kathetersystem gemäß Anspruch 1, wobei der Führungskatheter (11) eine distale axiale Öffnung umfasst und wobei, in der ersten Konfiguration des Katheters (11), das Längsführungslumen in der axialen Öffnung endet.

3. Kathetersystem gemäß Anspruch 1 oder 2, wobei der Führungskatheter (11) eine Führungseinrichtung (14) umfasst, welche im wesentlichen die seitliche Öffnung (13) in der ersten Konfiguration des Katheters (11) blockiert und welche zur Führung eines Drahtes durch die seitliche Öffnung (13) in der zweiten Konfiguration des Katheters (11) angeordnet ist.

4. Kathetersystem gemäß einem der vorstehenden Ansprüche, das ferner einen Schneidedraht (19) mit einer Schneidespitze (20) an seinem distalen Ende umfasst, wobei der Schneidedraht (19) durch den Führungskatheter (11) vorschiebbar ist, um so seine Schneidespitze (20) durch die unblockierte seitliche Öffnung (13) des Führungskatheters (11) vorragen zu lassen.

5. Kathetersystem gemäß Anspruch 3 oder 4, wobei die Führungseinrichtung eine Verschlussklappe (14) umfasst, welche klappbar an die seitliche Öffnung (13) gebunden ist und welche ein proximales freies Ende besitzt.

6. Kathetersystem gemäß Anspruch 5, wobei die Verschlussklappe (14) zwischen einer Position, die im Wesentlichen die seitliche Öffnung (13) des Führungskatheters (11) blockiert und einer Position, worin die Verschlussklappe (14) für das Führen eines Drahtes durch die seitliche Öffnung (13) schräggestellt ist, bewegbar ist.

7. Kathetersystem gemäß einem der vorhergehenden Ansprüche, das außerdem einen rotierenden Positionsindikator (21) umfasst, der auf dem Führungskatheter (11) angeordnet ist.

8. Kathetersystem gemäß Anspruch 7, wobei der rotierende Positionsindikator (21) ein Ultraschall reflektierendes Material an einer vorbestimmten Position relativ zu der seitlichen Öffnung (13) des Führungskatheters (11) umfasst.

9. Kathetersystem gemäß Anspruch 8, wobei das Ultraschall reflektierende Material (21) mindestens teilweise die seitliche Öffnung (13) des Führungskatheters (11) umringt.

10. Kathetersystem gemäß einem der vorhergehenden Ansprüche, das außerdem einen aufblasbaren Ballon (23)zum Fixieren der Position des distalen Endes (12) des Führungskatheters (11) in dem Körpergefäß umfasst.

11. Kathetersystem gemäß einem der Ansprüche 7-9, das außerdem einen Positionierungskatheter (25; 25') zur Einführung in ein anderes menschliches Körpergefäß umfasst und das einen auf den rotierenden Positionsindikator (21) ansprechenden Detektor (22) besitzt.

12. Kathetersystem gemäß Anspruch 11, wobei der Positionierungskatheter ein Ultraschallkatheter (25; 25') ist, der einen Ultraschallfrequenztransducer (22) zur Überwachung einer Positionierung der seitliche Öffnung (13) des Führungskatheters (11) umfasst, wenn er in das Körpergefäß eingeführt ist.

13. Kathetersystem gemäß Anspruch 11 oder 12, wobei der Positionierungskatheter (25) zwei parallele Lumina (26, 28; 205, 210) umfasst, von denen eine (26; 205) eine axial gerichtete distale Öffnung (27) besitzt und die andere von ihnen (28; 210) eine seitlich gerichtete Öffnung (29; 33) besitzt.

14. Kathetersystem gemäß Anspruch 13, wobei die seitlich gerichtete Öffnung (29) eine distale Öffnung der anderen (28) der parallelen Lumina ist.

15. Kathetersystem gemäß Anspruch 14, das außerdem eine Verschlussklappe (34) umfasst, die mit einer distalen Kante der seitlich gerichteten Öffnung (29) verbunden ist, wobei die Verschlussklappe auf die gegenüberliegende Seite der anderen (210) der parallelen Lumina geklappt ist und eine freies Ende, proximal zu der distalen Kante, besitzt.

16. Kathetersystem gemäß Anspruch 15, wobei die Verschlussklappe (34) flexibel zu einer Position ist, die im wesentlichen die seitlich gerichtete Öffnung (33) der anderen (210) der parallelen Lumina bedeckt.

17. Kathetersystem gemäß Anspruch 16, wobei die andere (210) der parallelen Lumina auch eine axial gerichtete distale Öffnung besitzt.

18. Kathetersystem gemäß Anspruch 12-17, wobei der Ultraschallfrequenztransducer (22) seitlich im Wesentlichen in der Richtung der seitlich gerichteten Öffnung (29) des Ultraschallkatheters (25) gerichtet ist.

19. Kathetersystem gemäß Anspruch 18, wobei der Ultraschallfrequenztransducer (22) distal zu der seitlich gerichteten Öffnung (29) positioniert ist.

20. Kathetersystem gemäß Anspruch 11-19, das außerdem einen aufblasbaren Ballon (30) zum Fixieren der Position des distalen Endes des Positionierungskatheters (25; 25') in dem anderen Körpergefäß umfasst.

21. Kathetersystem gemäß einem der vorhergehenden Ansprüche, wobei der Führungskatheter (11) außerdem ein zweites Längslumen umfasst, das eine axial gerichtete distale Öffnung besitzt.

## Revendications

1. Système de cathéter comprenant :
un cathéter de guidage (11) pour l'introduction dans un vaisseau corporel humain, ledit cathéter (11) comprenant :
une ouverture latérale distale (13), et
une lumière de guidage longitudinale,
**caractérisé en ce que** ledit cathéter (11) pendant ladite introduction a une première configuration dans laquelle ladite ouverture latérale est essentiellement bloquée, et
dans lequel ledit cathéter (11) lorsqu'il est positionné dans ledit vaisseau corporel, a une seconde configuration dans laquelle ladite ouverture latérale est débloquée et ladite lumière se termine dans ladite ouverture latérale, un transfert de la première à la seconde configuration étant contrôlable par un mouvement longitudinal d'un fil s'étendant à travers ladite lumière longitudinale.

2. Système de cathéter selon la revendication 1, dans lequel ledit cathéter de guidage (11) comprend en outre une ouverture axiale distale et dans lequel, dans ladite première configuration du cathéter (11), ladite lumière de guidage longitudinale se termine dans ladite ouverture axiale.

3. Système de cathéter selon la revendication 1 ou 2, dans lequel ledit cathéter de guidage (11) comprend en outre des moyens de guidage (14), qui bloquent essentiellement ladite ouverture latérale (13) dans la première configuration du cathéter (11) et qui sont agencés pour guider un fil à travers ladite ouverture latérale (13) dans la seconde configuration du cathéter (11).

4. Système de cathéter selon l'une quelconque des revendications précédentes, comprenant en outre un fil de coupe (19) ayant une pointe de coupe (20) au niveau de son extrémité distale, ledit fil de coupe (19) pouvant avancer à travers le cathéter de guidage (11) afin de faire faire saillie à sa pointe de coupe (20) à travers l'ouverture latérale (13) débloquée du cathéter de guidage (11).

5. Système de cathéter selon la revendication 3 ou 4, dans lequel les moyens de guidage comprennent un volet (14) qui est raccordé de manière articulée au niveau de l'ouverture latérale (13) et qui a une extrémité libre proximale.

6. Système de cathéter selon la revendication 5, dans lequel le volet (14) est mobile entre une position bloquant essentiellement l'ouverture latérale (13) du cathéter de guidage (11) et une position dans laquelle le volet (14) est incliné pour guider un fil à travers ladite ouverture latérale (13).

7. Système de cathéter selon l'une quelconque des revendications précédentes, comprenant en outre un indicateur de position rotatif (21) agencé sur le cathéter de guidage (11) .

8. Système de cathéter selon la revendication 7, dans lequel l'indicateur de position rotatif (21) comprend un matériau de réflexion ultrasonore au niveau d'une position prédéterminée par rapport à l'ouverture latérale (13) du cathéter de guidage (11).

9. Système de cathéter selon la revendication 8, dans lequel le matériau de réflexion ultrasonore (21) encercle au moins partiellement l'ouverture latérale (13) du cathéter de guidage (11).

10. Système de cathéter selon l'une quelconque des revendications précédentes, comprenant en outre un ballonnet gonflable (23) pour fixer la position de l'extrémité distale (12) du cathéter de guidage (11) dans le vaisseau corporel.

11. Système de cathéter selon l'une quelconque des revendications 7 à 9, comprenant en outre un cathéter de positionnement (25 ; 25') pour l'introduction dans un autre vaisseau corporel humain et ayant un détecteur (22) sensible à l'indicateur de position rotatif (21).

12. Système de cathéter selon la revendication 11, dans lequel le cathéter de positionnement est un cathéter à ultrasons (25 ; 25') comprenant un émetteur ultrasonore (22) pour surveiller un positionnement de l'ouverture latérale (13) du cathéter de guidage (11) lorsqu'il est introduit dans ledit vaisseau corporel.

13. Système de cathéter selon la revendication 11 ou 12, dans lequel le cathéter de positionnement (25) comprend deux lumières parallèles (26, 28 ; 205, 210), dont l'une (26 ; 205) a une ouverture distale (27) dirigée de manière axiale et l'autre (28 ; 210) a une ouverture (29 ; 33) dirigée de manière latérale.

14. Système de cathéter selon la revendication 13, dans lequel l'ouverture (29) dirigée de manière latérale est une ouverture distale de l'autre lumière (28) desdites lumières parallèles.

15. Système de cathéter selon la revendication 14, comprenant en outre un volet (34) raccordé à un bord distal de l'ouverture (29) dirigé de manière latérale, ledit volet étant incliné vers le côté opposé de l'autre lumière (210) desdites lumières parallèles et ayant une extrémité libre proximale par rapport audit bord distal.

16. Système de cathéter selon la revendication 15, dans lequel le volet (34) est flexible dans une position recouvrant sensiblement l'ouverture (33) dirigée de manière latérale de l'autre lumière (210) desdites lumières parallèles.

17. Système de cathéter selon la revendication 16, dans lequel l'autre lumière (210) desdites lumières parallèles a également une ouverture distale dirigée de manière axiale.

18. Système de cathéter selon les revendications 12 à 17, dans lequel l'émetteur ultrasonore (22) est dirigé latéralement sensiblement dans la direction de l'ouverture (29) dirigée de manière latérale du cathéter ultrasonore (25).

19. Système de cathéter selon la revendication 18, dans lequel l'émetteur ultrasonore (22) est positionné de manière distale par rapport à l'ouverture (29) dirigée de manière latérale.

20. Système de cathéter selon les revendications 11 à 19, comprenant en outre un ballonnet gonflable (30) pour fixer la position de l'extrémité distale du cathéter de positionnement (25 ; 25') dans ledit autre vaisseau corporel.

21. Système de cathéter selon l'une quelconque des revendications précédentes, dans lequel le cathéter de guidage (11) comprend en outre une seconde lumière longitudinale ayant une ouverture distale dirigée de manière axiale.
